# EUROPEAN PATENT APPLICATION

(11) **EP 2 098 126 A1**
(43) Date of publication of application: **09.09.2009**
(21) Application number: 08152222.9
(22) Date of filing: 03.03.2008
(51) Int. Cl.: A23L 1/164, A23G 3/42

(54) **Carbohydrate Bar**

(71) Applicant: Nestec S.A., 1800 Vevey (CH)
(72) Inventor: Jeukendrup, Asker, Birmingham, West Midlands B17 0TL (GB); Stellingwerff, Trent, 1000 Lausanne 26 (CH); Zaltas, Eric, 1806 St-Legier (CH)
(74) Representative: Marquardt, Ulf

(57) **Abstract**

The present invention generally relates to the field of nutrition, in particular performance nutrition. In particular, the present invention relates to a novel carbohydrate bar comprising glucose and fructose in a ratio in the range of 3:1 to 1:1. The carbohydrate bar of the present invention can be used to treat or prevent problems with the gastrointestinal tract while allowing for an enhanced blood sugar maintenance and/or an increased exogenous carbohydrate oxidation.

## Description

The present invention generally relates to the field of nutrition, in particular performance nutrition. In particular, the present invention relates to a novel carbohydrate bar.

It is well established that carbohydrate ingestion during exercise improves endurance performance during prolonged (>2 h) exercise, but also during shorter duration exercise (Coyle EF, J Sports Sci 22: 39-55, 2004; Gisolfi CV. Med Sci Sports Exerc 24: 679-687, 1992, Eukendrup AE and Jentjens R. Sports Med 29: 407-424, 2000).

Endurance exercise, and in particular running, has been associated with gastrointestinal (GI) disturbances which can ultimately affect performance (for reviews see: (Brouns F, et al., Int J Sports Med 8: 175-189, 1987, Gisolfi CV. News Physiol Sci 15: 114-119, 2000, Peters HP, et. Al., Gut 48: 435-439, 2001). In fact, a 30 to 50% prevalence of exercise-related adverse GI symptoms has been reported among endurance athletes.

A variety of symptoms may occur during exercise, which may be attributed to disorders of the upper (esophagus and stomach) or lower (small bowel and colon) GI tract. Upper GI symptoms include reflux, nausea, bloating, and upper abdominal cramping. Lower GI complaints comprise lower abdominal cramping, the urge to defecate, increased frequency of bowel movements, flatulence and diarrhoea. Many of these symptoms have been shown to be exacerbated with fluid intake, and specifically carbohydrate intake, which slows gastric emptying and can lead to significant GI disturbances.

In line with these negative GI issues related to a high carbohydrate intake, and given the fact that previous research has shown that a single source of carbohydrate can only be oxidized at a maximum of 1g/min or 60g/h (Jeukendrup AE and Jentjens R., Sports Med 29: 407-424, 2000.), in 2000 the American College of Sports Medicine (ACSM) current recommendations for carbohydrate intake during exercise is 30-60 g CHO/ h (American College of Sports Medicine, American Dietetic Association, and Dietitians of Canada. Med Sci Sports Exerc 32: 2130-2145, 2000).

Recently, a series of studies has shown that a combination of multiple carbohydrate sources, such as glucose and fructose, or maltodextrin and fructose, in the form of a sports drink can result in a higher total carbohydrate delivery during exercise than a single carbohydrate source, which results in a 30 to 50% greater efficiency of use and exogenous carbohydrate oxidation (Jentjens RL and Jeukendrup AE, Br J Nutr 93: 485-492, 2005; Jeukendrup AE,. Nutrition 20: 669-677, 2004; Jeukendrup AE and Jentjens R., Sports Med 29: 407-424, 2000; Jeukendrup AE, et al., J Appl Physiol, 2005). When subjects consumed a combination of glucose and fructose this resulted in significantly greater endurance performance compared to an isocaloric amount of glucose alone (Currell K and Jeukendrup AE. Med Sci Sports Exerc 40: 275-281, 2008). In this study subjects were able to complete a 1-hour time trial, after 2 hours of moderate intensity cycling, 8% faster when consuming a combination of glucose and fructose, compared to glucose alone.

This effect was, however, only observed when the carbohydrates were consumed in large amounts and in the form of a sports drink. In order to ingest the required amounts of carbohydrates it was necessary to consume large amounts of liquids.

These controlled laboratory findings are in contrast to real-life competition, during which athletes tend to drink smaller amounts of fluid, and ingest only small amounts of carbohydrate for fear of GI upset. No studies on the tolerance of multiple carbohydrate sources in real-life situations have been conducted so far.

Further, during exercise the ingestion of large amounts of liquids and carbohydrates causes an increase in osmolarity, which causes a decrease in gastric (stomach) emptying, and thus, a decrease in fluid delivery. This decreased gastric emptying during exercise is not wanted, since this causes bloating and an uncomfortable feeling of too much liquid sloshing in the stomach.

Additionally, consuming fructose is known to be especially distressful to GI problems with athletes (Ledochowski M, et al., Scand J Gastroenterol 36: 367-371, 2001; Mitsui T, et al., J Sports Med Phys Fitness 41: 121-123, 2001.). In short, the prevalence of GI disturbances is high among endurance athletes and seems to be related to CHO intake during exercise, in particular when consuming fructose alone.

Consequently, based on these findings and in view of problems with the GI tract of athletes, the consumption of fructose alone cannot be recommended.

Based on this prior art it was the object of the present invention to provide the art with a formulation that can provide athletes very efficiently with carbohydrates and an increased rate of exogenous carbohydrate oxidation during exercise, while at the same time minimizing the risk of developing problems with the gastrointestinal tract.

The present inventors were surprised to find that they could achieve this object by a carbohydrate bar in accordance with claim 1 and by a use in accordance with claim 12.

While the subject matter of the present invention is primarily intended for athletes, it is, however, clear, that the carbohydrate bar of the present invention can be used by anybody in need of carbohydrate supply. For example, the bar of the present invention can be very well used as transportable food for people, who do not wish to carry too heavy provisions, for example during long term trips. The bar of the present invention may equally well be used by people to provide the body with carbohydrates for example before or during an examination in school or at the university.

The present inventors have investigated the gastric tolerance of a carbohydrate bar delivering a mixture of glucose and fructose at relatively high rates during a real life situation of either running or cycling outdoors.

It was found that an intake of even 90 grams of carbohydrate per hour in the form of a carbohydrate bar, as well as 30-60 g CHO/ h as recommended by the ACSM, is well tolerable if ingested as carbohydrate mixture comprising glucose and fructose in a ratio in the range of 3:1 to 1:1, preferably 2:1 in the form of a bar.

It was surprisingly found that the ingestion of 90 grams/h of the above carbohydrate mixture will allow to keep the distress of the GI-tract of athletes minimal, and at about a rate of ∼15% of subjects. Furthermore it was found that the minimal GI distress was not increased as compared with an intake of 60 grams of a carbohydrate mixture comprising glucose and fructose in a ratio in the range of 3:1 to 1:1, preferably 2:1 per hour.

Consequently, one embodiment of the present invention is a carbohydrate bar comprising a carbohydrate fraction comprising glucose and fructose in a ratio in the range of 3:1 to 1:1.

A carbohydrate bar is a food product, preferably in bar form, which comprises at least one carbohydrate source, and preferably a multiple carbohydrate source such as the combination of glucose and fructose, or maltodextrin and fructose. The shape of the carbohydrate bar is not essential, it does not have to be rectangular, it may equally well have other shapes, for example round or triangular..

The carbohydrate bar is preferably a dried food product. It may comprise a crust and a filling.

The carbohydrate bar of the present invention contains preferably less than 30 %, preferably less than 20 %, even more preferred less than 10 %, in particular preferred less than 8 % moisture.

The carbohydrate bar may contain a grain or grains, nuts, possibly dried fruit, fruit paste, sweeteners and other ingredients. These ingredients can be mixed with a binder such as a sugar syrup or shortening and compressed into bars or slabs which may be later cut to a desired size. Depending on the carbohydrate bar's composition it may be mixed, formed, extruded, enrobed and/or baked prior to packaging and sale.

For achieving good results in compressed foods, glycerine and/or other polyhydric alcohols may be added to the bar to yield better mold release and bonding strength of the overall carbohydrate bar.

The multiple carbohydrate source contains fructose and glucose in a digestible form. Glucose and/or fructose may be provided in the form of fructogenic and/or glucogenic carbohydrates. Fructogenic carbohydrates are carbohydrates which upon theoretical total hydrolysis release at least one fructose molecule. Glucogenic carbohydrates are carbohydrates which upon theoretical total hydrolysis release at least one glucose molecule. Consequently, a carbohydrate can be both, glucogenic and fructogenic (e.g., saccharose). For example in performance bars, glucogenic sources may be also extruded cereal pieces, cereal flakes, flours and starches.

Hence, the carbohydrates may comprise or consist of monosaccharides, such as glucose or fructose as basic carbohydrate units. The monosaccharides may represent a part of disaccharides, such as sucrose, lactose, maltose or cellobiose. The monosaccharides such as glucose or fructose may also represent a part of oligosaccharides or polysaccharides. Preferred carbohydrate sources for the present invention are maltodextrins and/or dextrose.

The carbohydrate source may additionally comprise indigestible carbohydrates, in particular fibers.

The carbohydrate fraction of the bar may comprise at least 30 % glucose and fructose, preferably at least 50 % glucose and fructose more preferably at least 85 % glucose and fructose. In one embodiment of the present invention the carbohydrate fraction of the bar provides at least 50 %, preferably at least 70 % of the energy of the bar.

The carbohydrate bar of the present invention comprises a carbohydrate fraction and, optionally, a protein fraction and/or a fat fraction.

The presence of proteins and/or fats in the carbohydrate bar of the present invention has the advantage that this way it is possible to provide the athlete with a more complete nutrition during performance. Furthermore, the presence of proteins allows producing a carbohydrate bar with a modified taste.

As protein source, any suitable dietary protein may be used, for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred.

The proteins may be intact or hydrolysed or a mixture of intact and hydrolysed proteins. It may be desirable to supply partially hydrolysed proteins (degree of hydrolysis between 2 and 20%), for example for athletes believed to be at risk of developing cows' milk allergy. Additionally, in generally at least partially hydrolysed proteins are easier and faster to metabolize by the body. This is in particular true for amino acids. Consequently, it is further preferred if the carbohydrate bar of the present invention contains single amino acids, most preferred are essential amino acids. In one embodiment the carbohydrate bar of the present invention contains amino acids such as L-leucine, L-valine and/or L-isoleucine.

If the composition includes a fat source, the fat source has the advantage that for example an improved mouth feel can be achieved. Any fat source is suitable. For example animal or plant fats may be used. To increase the nutritional value, n3-unsaturated and n6-unsaturated fatty acids may be comprised by the fat source. The fat source may also contain long chain fatty acids and/or medium chain fatty acids. For example, milk fat, canola oil, almond butter, peanut butter, corn oil and/or high-oleic acid sunflower oil may be used.

Preferably, the bar contains less than 40 g protein per 100g bar and/or less than 20 g fat per 100g bar.

The carbohydrate bar may also contain minerals and micronutrients such as trace elements and vitamins in accordance with the recommendations of Government bodies such as the USRDA

The carbohydrate bar of the present invention may contain vitamins, such as Vitamin C, Vitamin E, Vitamin B12, Niacin, Vitamin B6, folic acid, biotin, panthotenic acid, Vitamin B₂ and/or Vitamin B6, preferably in amounts that correspond to at least 10 % of the recommended daily dose.

The presence of vitamins may contribute to the effectiveness of the bar and may further protect the athlete. For example, the presence of vitamin C will help to protect against catching a common cold.

The bar may also comprise electrolytes and/or minerals, such as sodium, potassium, calcium, iron, magnesium or zinc.

These compounds may be helpful to replenish the body with compounds that the person is constantly loosing due to the generation of sweat during exercise. They may also help to prevent the generation of post-exercise sore muscles.

The carbohydrate bar of the present invention may further contain one or more compounds selected from the group consisting of aroma compounds, fiber, caffeine, conservatives, guarana, acidifying agents, binding agents, gel building material, water, fruit juice, fruits, antioxidants, colouring agents.

These agents may improve the carbohydrate bar of the present invention with respect to many properties, such as taste, consistency, colour, and stability during storage, digestibility, and many more that are known to those of skill in the art.

The energy density of the bar is not critical for its effectiveness. However, a high energy density has the advantage that less food needs to be ingested to replenish carbohydrates as fuel to the body. Consequently, high energy densities are preferred for the bar of the present invention.

One embodiment of the present invention is **characterized in that** the carbohydrate bar has an energy density of 800-2200 kJ/100g, preferably 1000-2000 kJ/100g, most preferred 1200-1800kJ/100g.

To be easily consumable - for example during a competition or in between competitions - the serving size of the bar of the present invention is preferably relatively small. Preferably, the carbohydrate bar has a serving size of 10-200 g, preferably 20-1 00g, most preferred 50-80g.

Alternatively, the bar of the present invention may also be provided as bite size bars with a serving size between 3 and 15g, preferably between 5 and 10g. This way, the carbohydrate uptake can precisely be adjusted to the needs of an athlete.

A typical carbohydrate bar of the present invention may comprise the following percentages of daily values (DV) based on a 2000 calorie diet: Between 4 and 6 % total fat, including between 1 and 3 % saturated fats, between 5 and 9 % sodium, between 0,5 and 1,5 % potassium, between 12 and 16 % carbohydrates, including between 5 and 10 % glucose and fructose, and between 10 and 14 % proteins.

Additionally it may comprise between 80 and 120 % DV vitamin C, between 20 and 30 % DV calcium, between 25 and 35 % DV iron, between 80 and 120 % DV vitamin E, between 80 and 120 % DV thiamin, between 80 and 120 % DV riboflavin, between 80 and 120 % DV niacin, between 80 and 120 % DV vitamin B6, between 80 and 120 % DV folate, between 80 and 120 % DV vitamin B12, between 80 and 120 % DV biotin, between 80 and 120 % DV pantothenic acid, between 20 and 30 % DV phosphorus, between 20 and 30 % DV magnesium, between 25 and 35 % DV zinc, between 25 and 35 % copper, and between 15 and 25 % DV chromium.

The carbohydrate bar of the present invention may be used for example as a food product, as a food additive or as a nutraceutical.

In a preferred embodiment of the present invention the carbohydrate bar of the present invention is used for the preparation of a food product or as a food product to provide an increased performance, in particular endurance performance.

The formulation of this carbohydrate bar of the present invention may also be used to provide an increased carbohydrate delivery without any increased gastrointestinal disorders and/or to treat or prevent problems with the gastrointestinal tract.

The combination of glucose and fructose in the carbohydrate bar of the present invention allows it that the high carbohydrate content of the bar of the present invention is well tolerated by the body, so that problems of the gastrointestinal tract that one would normally expect after a high level of carbohydrate intake during exercise are at least partially avoided. Also the gastrointestinal tolerance for carbohydrates, in particular the glucose/fructose mixture of the present invention will be increased.

The problems with the gastrointestinal tract as mentioned above are not particularly limited but are preferably selected from the group consisting of upper abdominal problems such as reflux, heartburn, bloating, upper abdominal cramps, vomiting, nausea; lower abdominal problems such as intestinal cramps, flatulence, urge to defecate, left abdominal pain, right abdominal pain, loose stool, diarrhoea; or systemic problems such as dizziness, headache, muscle cramp or urge to urinate.

For exercise in general, but in particular for competitive exercise it is essential that the body has blood sugar available for the muscles to burn at all times. In particular at the end of a race it must be avoided, or the athlete will run out of energy. The subject matter of the present invention is well suited to prevent this. According to one embodiment of the present invention the carbohydrate bar of the present invention can be used to allow for an enhanced blood sugar maintenance late in exercise.

The carbohydrate bar of the present invention cannot only secure a long lasting blood sugar maintenance, it can also be used to provide an increased exogenous carbohydrate oxidation. Increasing exogenous carbohydrate oxidation was found to be in particular increased if the carbohydrate uptake of a person is greater than 1 g/min, preferably greater than 1.1 g/min even more preferred greater than 1.2 g/min.

Consequently, surprisingly, optimal exogenous carbohydrate oxidation is reached at a carbohydrate ingestion above the theoretical threshold once thought for carbohydrate oxidation (Jeukendrup AE and Jentjens R., Sports Med 29: 407-424, 2000).

This way, the energy delivery from carbohydrates during physical exercise can be maximised.

Further, the carbohydrate bar of the present invention may be used to provide faster energy delivery, in particular to working muscles, and/or to provide more sustained energy to muscles. Both effects will contribute to an optimal performance of an athlete.

Finally, the carbohydrate bar of the present invention may also be used to treat or prevent symptoms of fatigue and/or to improve cycling cadence, for example measured in revolutions per minute and/or to decrease ratings of perceived exertion (RPE).

The present inventors found that the above listed uses can be successfully carried out with any amounts of carbohydrates comprising glucose and fructose in a ratio of 3:1 to 1:1 to be ingested

However, best results were obtained, when the carbohydrate bar was used in an amount that corresponds to an ingestion of at least 30g CHO/h, preferably at least 50g CHO/h, more preferably at least 65g CHO/h and most preferably between 80g CHO/h and 110g CHO/h.

The inventors have found that the higher the amount of carbohydrates ingested per hour is, the more the exogenous carbohydrate oxidation can be increased. A maximum of exogenous carbohydrate oxidation appears to be reached when the bar of the present invention is consumed so that 100g - 150g carbohydrates are consumed per hour, preferably 110g - 130 g carbohydrates per hour and most preferred 115g-125g carbohydrates per hour.

It is clear to those skilled in the art that they can freely combine all features of the present invention disclosed herein without departing from the subject matter as disclosed.

Further features and advantages of the present invention are apparent from the following Examples and Figures.

Figure 1 shows the results of the study of Example 2.

Figure 2 shows the results of the study of Example 3.

### Example 1: Bar formulation

A typical formulation for a bar in accordance with the present invention is presented in table form below:

| **Ingredient** | Amount (%) | Total Carbs in Bar | Sugars (Glucogenic) in Bar | Sugars (Fructogenic) in Bar | Other Carbs (glucogenic) in Bar |
|---|---|---|---|---|---|
| Maltodextrin | 12.933% | 12.22 | 0.75 | 0.00 | 11.47 |
| Milk Protein Isolate | 8.979% | 0.01 | 0.01 | 0.00 | 0.00 |
| Soy Protein Isolate | 0.500% | 0.01 | 0.01 | 0.00 | 0.00 |
| Rice Flour | 5.273% | 4.01 | 0.00 | 0.00 | 3.74 |
| Flour Peanut | 0.100% | 0.02 | 0.01 | 0.00 | 0.00 |
| Oat Bran | 14.360% | 6.98 | 0.00 | 0.00 | 4.77 |
| Rice Crisps | 8.985% | 7.01 | 0.31 | 0.00 | 5.84 |
| Crystalline Fructose | 6.800% | 6.77 | 0.00 | 6.77 | 0.00 |
| Evap Cane Juice | 33.150% | 27.85 | 13.92 | 13.92 | 0.00 |
| Syrup | | | | | |
| salt | 0.300% | | | | |
| Glycerine, | 1.000% | 0.04 | 0.00 | 0.00 | 0.00 |
| Almond Butter | 3.278% | 0.25 | 0.16 | 0.00 | 0.08 |
| Vanilla | 1.234% | 0.72 | 0.19 | 0.19 | 0.32 |
| Vitamin/Mineral/Ami | 3.108% | 0.20 | 0.00 | 0.00 | 0.20 |
| no Premix | | | | | |
| **Total** | **100.000%** | **66.08** | **15.36** | **20.88** | **26.45** |

| | | |
|---|---|---|
| Glucogenic Carbs | 41.80 | 0.67 |
| Fructogenic Carbs | 20.88 | 0.33 |
| Glucogenic Carbs/Fructogenic Carbs ratio | 2.00 | |

### The bar is produced as follows:

All wet ingredients are mixed together (syrup, glycerine, almond butter and flavours) at 50°C. Separetaly, dry ingredients are mixed together, then the wet slurry is added to the dry mix and the mass is mixed for 2 to 5 minutes under high shear. The dough is slabed and cut into bar shape before packing.

### Example 2:

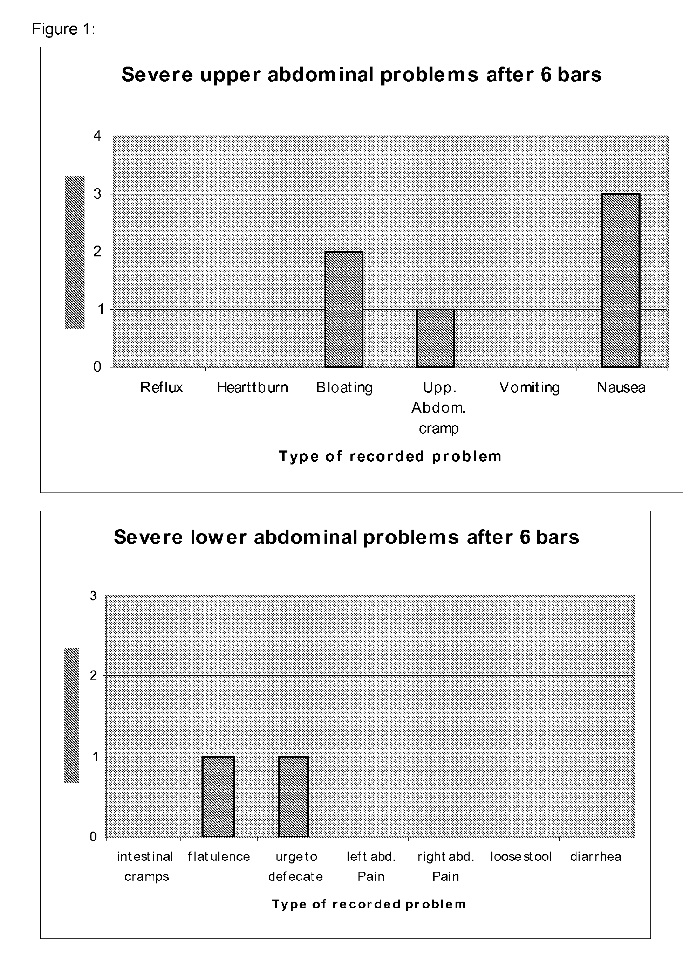

The tolerance of a carbohydrate (in the form of glucose and fructose in the ratio of 2:1) intake of 90 g /h in the form of bars was tested. The bars were prepared according to Example 1. 14 cyclists cycled 68 miles at an average speed of 19.5 miles/hour and consumed 6 bars (app. ½ bar every 15 minutes). Each rider completed a questionnaire immediately after the ride with questions about gastrointestinal complaints during the ride. Results are shown in Figure 1.
While with a composition comprising only glucose consumed at a rate of about 90g glucose/hour all cyclists would be expected to suffer from severe gastrointestinal problems, the bars of the present invention allowed to reduce these problems significantly.

### Example 3:

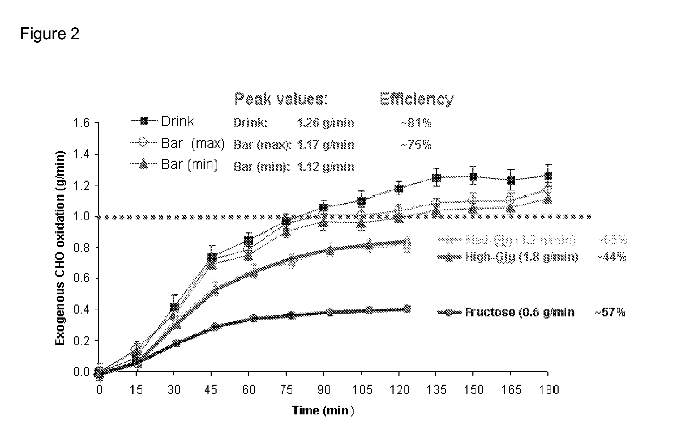

The exogenous carbohydrate oxidation rates of a bar formulated with a 2:1 glucose and fructose ratio were investigated versus a sports drink formulated also with a 2:1 ratio of glucose and fructose, as compared to previous research data of glucose and fructose alone. The bars were prepared according to Example 1. 8 male cyclists rode in the lab for 3 hrs at ∼60% VO2peak, and consumed ½ bar every 15min, or the equivalent in sports drink, so that the bar and sports drink resulted in a consumption of 1.55g CHO/min. Blood and breath samples were collected throughout the 3 hrs to measure exogenous carbohydrate oxidation rates of the bar vs. the sports drink. Each subject also completed a questionnaire immediately after the ride with questions about gastrointestinal complaints during the ride. Results are shown in Figure 2. As shown, there was no statistical difference in carbohydrate oxidation between either the sports drink or bar form, showing the bar is as effective in providing carbohydrate at a high level of efficiency (∼75%) as the multi-carbohydrate source sports drink. The peak oxidation of the multi-carbohydrate sources (2:1 glucose:fructose) bar and drink was ∼1.2g/min, which is considerably higher than achieved with a single carbohydrate source (glucose alone at ∼0.8g/min and fructose along at ∼0.4g/min).

## Claims

1. Carbohydrate bar comprising a carbohydrate fraction comprising glucose and fructose in a ratio in the range of 3:1 to 1:1.

2. Carbohydrate bar in accordance with claim 1, **characterized in that** the carbohydrate fraction of the bar comprises at least 30 % glucose and fructose, preferably 50 % glucose and fructose more preferably at least 85 % glucose and fructose.

3. Carbohydrate bar in accordance with one of the preceding claims, **characterized in that** the carbohydrate fraction of the bar provides at least 50 %, preferably at least 70 % of the energy of the bar.

4. Carbohydrate bar in accordance with one of the preceding claims, **characterized in that** the carbohydrate fraction of the bar comprises dextrose and/or maltodextrins.

5. Carbohydrate bar in accordance with one of the preceding claims, **characterized in that** it contains less than 40 g protein per 100g bar and/or less than 20 g fat per 100g bar.

6. Carbohydrate bar in accordance with one of the preceding claims, **characterized in that** it further contains vitamins, such as Vitamin C, Vitamin E, Vitamin B12, Niacin, Vitamin B6, folic acid, biotin, panthotenic acid, Vitamin B₂ and/or Vitamin B6, preferably in amounts that correspond to at least 10 % of the recommended daily dose.

7. Carbohydrate bar in accordance with one of the preceding claims, **characterized in that** in further comprises electrolytes and/or minerals, such as sodium, potassium, calcium, iron, magnesium or zinc.

8. Carbohydrate bar in accordance with one of the preceding claims, **characterized in that** it further contains amino acids such as I-leucine, L-valine and/or L-isoleucine.

9. Carbohydrate bar in accordance with one of the preceding claims, **characterized in that** it has an energy density of 800-2200 kJ/100g.

10. Carbohydrate bar in accordance with one of the preceding claims, **characterized in that** it has a serving size of 10-200 g, preferably 20-1 00g, or 3 - 15g, preferably 5 - 10g.

11. Carbohydrate bar in accordance with one of the preceding claims, **characterized in that** it further comprises one or more compounds selected from the group consisting of aroma compounds, fiber, caffeine, conservatives, guarana, acidifying agents, binding agents, gel building material, water, fruit juice, fruits, antioxidants, colouring agents.

12. Use of a carbohydrate bar in accordance with one of claims 1-11 as a food product or for the preparation of a food product to provide an increased performance, in particular endurance performance.

13. Use in accordance to claim 12 to further provide an increased gastrointestinal tolerance and/or to treat or prevent problems with the gastrointestinal tract.

14. Use in accordance with claim 13, wherein the problems with the gastrointestinal tract are selected from the group consisting of upper abdominal problems such as reflux, heartburn, bloating, upper abdominal cramps, vomiting, nausea; lower abdominal problems such as intestinal cramps, flatulence, urge to defecate, left abdominal pain, right abdominal pain, loose stool, diarrhoea; or systemic problems such as dizziness, headache, muscle cramp or urge to urinate.

15. Use in accordance with one of claims 12-14 to allow for an enhanced blood sugar maintenance late in exercise.

16. Use in accordance with one of claims 12-15 to provide an increased exogenous carbohydrate oxidation, to provide faster energy delivery, in particular to working muscles, and/or to provide more sustained energy to muscles.

17. Use in accordance with one of claims 12-16 to further treat or prevent symptoms of fatigue, to improve cycling cadence, for example measured in revolutions per minute and/or to decrease ratings of perceived exertion (RPE).

18. Use in accordance with one of claims 12-16, wherein the carbohydrate bar is used in an amount that corresponds to an ingestion of at least 30g CHO/h, preferably at least 50g CHO/h, more preferably at least 65g CHO/h and most preferably between 80g CHO/h and 110g CHO/h.
